# EUROPÄISCHE PATENTSCHRIFT

(11) **EP 1 350 842 B1**
(45) Veröffentlichungstag und Bekanntmachung des Hinweises auf die Patenterteilung: **15.02.2006**
(21) Anmeldenummer: 03006965.2
(22) Anmeldetag: 27.03.2003
(51) Int. Cl.: C12N 9/12, C12Q 1/48

(54) **Rekombinante terminale Deoxynukleotidyltransferase mit verbesserter Funktionalität**
Production and use of an improved terminal deoxynucleotidyl transferase
Préparation et utilisation d'une terminal déoxynucléotidyl transférase améliorée

(30) Priorität: 05.04.2002 DE 10215035
(43) Veröffentlichungstag der Anmeldung: 08.10.2003
(73) Patentinhaber: Roche Diagnostics GmbH, 68305 Mannheim (DE); F.HOFFMANN-LA ROCHE AG, 4070 Basel (CH)
(72) Erfinder: Mueller, Rainer, Dr., 82377 Penzberg (DE); Pajatsch, Markus, Dr., 81375 Muenchen (DE); Curdt, Ingo, Dr., 81377 Muenchen (DE); Sobek, Harald, Dr., 82377 Penzberg (DE); Schmidt, Manfred, 82377 Penzberg (DE); Suppmann, Bernhard, Dr., 82362 Weilheim (DE); Sonn, Kirsten, 82377 Penzberg (DE); Schneidinger, Bernd, Dr., 82069 Hohenschaeftlarn/Neufahrn (DE)
(74) Vertreter: Jung, Michael

(56) Entgegenhaltungen:
- SU-A- 805 631
- US-A- 5 037 756
- PANDEY V ET AL: "BIOCHEMISTRY OF TERMINAL DEOXYNUCLEOTIDYLTRANSFERASE AFFINITY LABELING AND IDENTIFICATION OF THE DEOXYNUCLEOSIDE TRIPHOSPHATE BINDING DOMAIN OF TERMINAL DEOXYNUCLEOTIDYLTRANSFERASE" JOURNAL OF BIOLOGICAL CHEMISTRY, Bd. 263, Nr. 8, 1988, Seiten 3744-3751, XP002256460 ISSN: 0021-9258
- TAKAHARA KAZUHIKO ET AL: "Alternative splicing of bovine terminal deoxynucleotidyl transferase cDNA." BIOSCIENCE BIOTECHNOLOGY AND BIOCHEMISTRY, Bd. 58, Nr. 4, 1994, Seiten 786-787, XP009018313 ISSN: 0916-8451
- PANDEY V ET AL: "PURIFICATION OF HIGH MOLECULAR MASS SPECIES OF CALF THYMUS TERMINAL DEOXYNUCLEOTIDYLTRANSFERASE" PREPARATIVE BIOCHEMISTRY, Bd. 17, Nr. 4, 1987, Seiten 359-378, XP009018512 ISSN: 0032-7484
- CHANG L M ET AL: "Proteolytic degradation of calf thymus terminal deoxynucleotidyl transferase." THE JOURNAL OF BIOLOGICAL CHEMISTRY. UNITED STATES 25 MAY 1982, Bd. 257, Nr. 10, 25. Mai 1982 (1982-05-25), Seiten 5700-5706, XP002256462 ISSN: 0021-9258
- KOIWAI O ET AL: "Isolation and characterization of bovine and mouse terminal deoxynucleotidyltransferase cDNAs expressible in mammalian cells." NUCLEIC ACIDS RESEARCH. ENGLAND 25 JUL 1986, Bd. 14, Nr. 14, 25. Juli 1986 (1986-07-25), Seiten 5777-5792, XP009018311 ISSN: 0305-1048
- DATABASE EMBL [Online] 9. März 1987 (1987-03-09) "Bovine mRNA for terminal deoxynucleotidyltransferase EC 2.7.7.31" retrieved from EBI Database accession no. X04122 XP002256463
- DATABASE EMBL [Online] 1. Januar 1988 (1988-01-01) "DNA nucleotidylexotransferase EC 2.7.7.31 from Bos taurus" retrieved from EBI Database accession no. P06526 XP002256464

## Beschreibung

Gegenstand der Erfindung ist eine rekombinante N-terminal verkürzte Terminale Deoxynukleotidyltransferase (TdT) aus Kalbsthymus, die unter bestimmten Bedingungen eine um das mindestens 20-fache gesteigerte Aktivität im Vergleich zur Volllängen-TdT aufweist, sowie deren Herstellung und Verwendung.

Die Terminale Deoxynukleotidyltransferase (TdT) ist ein hoch konserviertes Enzym aus Vertebraten, welches das Anhängen von 5'-Triphosphaten an die 3'-Hydroxylgruppe von Einzelstrang-DNA oder Doppelstrang katalysiert. Das Enzym agiert somit als Template-unabhängige Polymerase (Koiwai et al. (1986), Nucleic Acid Research 14 (14), 5777-5792). *In vivo* ist die TdT für die hohe Diversität von Immunglobulinen und T-Zellrezeptoren verantwortlich. Neben den natürlich vorkommenden Nukleosidtriphosphaten akzeptiert die TdT sowohl in der Regel radioaktiv markierte Triphopshate, als auch nicht-radioaktiv, z.B. Digoxigenin- oder Biotin, markierte Triphosphate. Die Akzeptanz von markierten Triphosphaten macht die TdT auch für Labor- und industrielle Anwendungen interessant (z.B. Oligotailing, *in situ* cell death detection bei Apoptosis-Nachweis).

*In vivo* unterliegt die Volllängen-TdT (Molekulargewicht ca. 58000 Da/520 Aminosäuren) einer schrittweise proteolytischen Degradation zu kleineren Fragmenten, die aber immer noch enzymatisch aktiv sind (Chang et al. (1982), *J. Biol. Chem.* 257(10): 5700-5706). Diese proteolytische Prozessierung generiert aus der 58 kDa-TdT vom N-Terminus her Peptide mit 56 kDa, 44 kDa und 42 kDa Molekulargewicht, die alle noch das aktive Zentrum besitzen. Weiterhin ist bekannt, dass das 42 kDa Peptid in ein aktives TdT-Fragment mit einem Molekulargewicht von 32 kDa degradiert wird, das sich wiederum aus 2 Peptiden, dem α-Peptid mit 8 kDa und dem β-Peptid mit 26 kDa, zusammensetzt. Dieses 32 kDa-Fragment ist auch die vorherrschende Form bei der Isolierung aus Kalbsthymus (Chang and Bollum (1986), *CRC Crit Rev Biochem* 21(1): 27-52).

Die Isolierung der TdT aus Kalbsthymus ist seit längerem bekannt (Deibel and Coleman (1979) *J. Biol. Chem.* 254(17): 8634-8640). Das von Deibel und Coleman beschriebene Verfahren ist zudem im Großrnaßstab wirtschaftlich, da Kalbsthymus ein kostengünstiger Rohstoff ist. Nachteilig bei diesem Verfahren ist jedoch, dass aufgrund der eingangs erwähnten proteolytischen Aktivierung eine homogene, bandenreine Aufreinigung sowie die Abtrennung der aktiven TdT-Fragmente von den inaktiven TdT-Fragmenten nicht möglich ist. Zudem ist die Isolierung von Einsatzstoffen aus bovinen Rohstoffen auf Grund der BSE-Problematik heutzutage möglichst zu vermeiden.
In Peterson et al. (1985, *J Biol Chem* 260 (19): 10495-502) und in US 5 037 756 (Bollum et al.) wird die Expression der humanen Volllängen-TdT bzw. diesen gegenüber geringfügig verkürzten TdT-Derivaten in *E.coli* beschrieben. Das exprimierte Protein wurde über einen Antikörper gegen TdT (aus Hase) im *E.coli*-Rohextrakt nachgewiesen und konnte über Immuno-Affinitätschromatographie als Volllängenprodukt aufgereinigt werden. Es ist jedoch zu erwarten, dass die Ausbeuten an TdT bei diesem Verfahren eher gering sind, zumindest sind keine Ausbeuten und Funktionsüberprüfungen beschrieben.

Chang et al. (*J. Biol. Chem*. 263 (25): 12509-12513) beschreibt 1988 die Expression der Volllängen-TdT aus Mensch in einem Baculovirussystem. Die Ausbeuten liegen hier bei etwa 10% des Gesamtproteingehaltes; das Enzym zeigt immunologische und enzymatische Aktivität. Nachteil dieses Verfahrens sind die oftmals niedrigeren Ausbeuten bei heterologer Proteinexpression im Baculovirussystem im Vergleich zu prokaryontischen Expressionssystemen und die höheren Herstellkosten bei einer Zellkulturfermentation.

In Yang et al (1995; *Nucleic Acids Research* 23 (11): 2041-2048) beschreiben die Expression der Volllängen-TdT aus Huhn in *E.coli.* Die rekombinante TdT ist hier kloniert in den Vektor pET16b und ist mit einem His-tag als Reinigungshilfe fusioniert. Die Analyse der rekombinanten TdT nach Isolierung ergab zwar das Volllängen-Produkt, jedoch eine um den Faktor 2 niedrigere Aktivität als die native Hühner-TdT. Nach Ansicht der Autoren ist als Grund für die niedrigere Aktivität der rekombinanten TdT entweder eine Störung der Aktivität durch den His-tag oder die fehlende postranslationale Modifizierung anzusehen.

Boule et al. beschreiben (1998; *Molecular Biotechnology* 10: 199-208) die Expression der Volllängen-TdT aus Maus in *E.coli.* Die Verwendung eines starken Promotors (T7-Promotor) zur Erhöhung der Expressionsrate führte zunächst zu einem hohen Anteil an inaktiv exprimierten Produkt ("inclusion bodies"). Dies konnten die Autoren im weiteren verhindern, indem sie die Wachstumstemperatur während der Induktionsphase extrem erniedrigten (15°C). Nachteilig dabei ist jedoch das verlangsamte Wachstum der *E.coli*-Zellen und die erhöhte Schaurnbildung bei diesen Fermentationstemperaturen.

Aufgabe der vorliegenden Erfindung ist daher, eine TdT in homogener Form mit ausreichender enzymatischen Aktivität zur Verfügung zu stellen.

Die der vorliegenden Erfindung zugrunde liegende Aufgabe wird gelöst durch ein verkürztes TdT-Enzym, welches N-terminal um 100 bis 160 Aminosäuren verkürzt ist und das im Vergleich zur Volllängen-TdT aus Kalbsthymus eine um das mindestens 20-fache gesteigerte enzymatische Aktivität in Cobalt-(Co²⁺)-Ionen haltigen Lösungen als im Zn/Mg-System aufweist. Besonders bevorzugt ist erfindungsgemäß eine N-terminal um 138 Aminosäuren verkürzte TdT. Entsprechende TdT-Derivate weisen in Co²⁺-Ionen haltigen Lösungen eine um das 20- bis 30-fache gesteigerte Enzymaktivität im Vergleich zu Volllängen-TdT aus Kalbsthymus im Zn/Mg-System auf. Die erfindungsgemäß verkürzte TdT weist ein Molekulargewicht (SDS-Page) von ca. 36 bis 46 kDa, bevorzugt zwischen 40 und 46 kDa, insbesondere zwischen 44 und 46 kDa auf.

Ein weiterer Gegenstand der Erfindung ist ein Verfahren zur rekombinanten Herstellung von TdT aus Kalbsthymus, welches durch folgende Schritte gekennzeichnet ist:
a) Transformation einer Wirtszelle mit einer Nukleinsäure, welche für ein N-terminal verkürztes TdT-Fragment kodiert und gegebenenfalls mit einer Nukleinsäure fusioniert ist, die für ein die anschließende Reinigung vereinfachendes Protein-tag kodiert,
b) Kultivierung der Wirtszelle zur Expression der rekombinanten verkürzten TdT unter für die jeweilige Wirtszelle geeigneten Kultivierungsbedingungen,
c) Isolierung der rekombinanten verkürzten TdT aus der Wirtszelle und
d) Einsatz und Überprüfung der rekombinanten verkürzten TdT in Funtkionsassay.

Als Wirtszelle im Sinne der Erfindung ist jede Wirtszelle zu verstehen, die in der Lage ist, Proteine in großen Mengen aktiv im Cytoplasma zu exprimieren. Üblicherweise handelt es sich hierbei um prokaryontische Zellen wie *Escherichia coli* oder *Bacillus subtilis,* aber auch Hefe- oder Pilzzellen wie *Pichia pastoris, Pichia methylotropha, Hansenula polymorpha, saccharomyces cerevisiae, Schizosaccharomyces pombae* u.a. oder *Aspergillus sp.* haben sich als geeignet erwiesen. Bevorzugt werden erfindungsgemäß *Escherichia coli*-Zellen eingesetzt.

Als Nukleinsäuren, welche für ein N-terminal verkürztes TdT-Fragment kodieren, kommen prinzipiell sämtliche von der cDNA der Kalbsthymus-TdT ableitbare Fragmente in betracht, soweit diese für ein Protein mit TdT-Aktivität kodieren. Insbesondere haben sich solche Nukleinsäuresequenzen erfindungsgemäß als vorteilhaft erwiesen, die für ein verkürztes TdT-Enyzm kodieren, welchem N-terminal bis zu 161 Aminosäuren gegenüber dem Wildtyp-Enzym fehlen. Als ganz besonders vorteilhaft haben sich erfindungsgemäß die folgenden Fragmente erwiesen: SEQ ID NO.: 7, SEQ ID NO.: 9 oder SEQ ID NO.: 11.

Das für ein verkürztes TdT-Protein kodierende Nukleinsäuremolekül kann für die Expression mit einer Nukleinsäuresequenz fusioniert sein, welche für ein Protein kodiert, das bei der anschließenden Reinigung der exprimierten TdT behilflich ist. Entsprechend geeignete Reinigungsprotein-tags bzw. die hierfür kodierenden DNA-Fragmente sind dem Fachmann prinzipiell geläufig. Neben dem (Poly)His-tag kommen beispielsweise Biotinylierungsproteine, Streptavidinbindende Proteine wie z.B. Streptacin® (Inst. für Bioanalytik, IBA, Göttingen/Deutschland), Maltose-bindende Proteine (z.B. US 5.643.758), GST- sowie HA-tags (z.B. US 5.654.176; WO98/17691) erfindungsgemäß in betracht.

Ein weiterer Gegenstand der Erfindung ist die Aufreinigung der verkürzten Terminale Transferase-Derivate aus dem Cytoplasma der Wirtszelle. Als Ausgangsmaterial für die Reinigung der rekombinanten Terminaler Transferase wurden dabei insbesondere *E. coli* K12 UT5600-Zellen verwendet, die das Terminale Transferase-Gen überexprimieren.

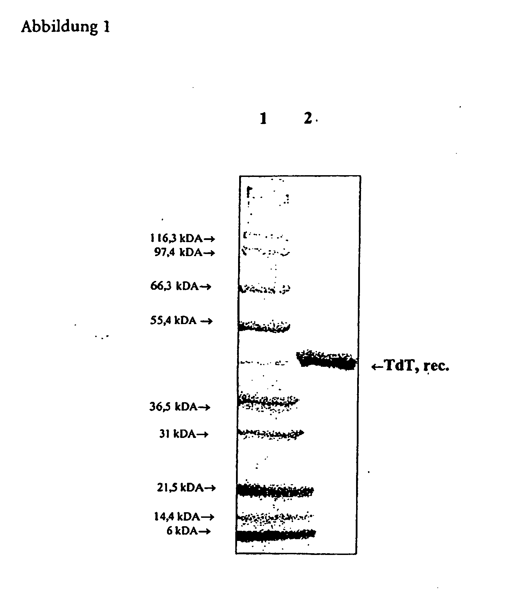

Die Reinigung der TdT erfolgt in der Regel bei 4 °C. Nach Zellaufschluss sowie Abtrennung der Nukleinsäuren, die prinzipiell nach bekannten Maßnahmen erfolgen können, schließen sich eine Folge von chromatographischen Schritten an. Erfindungsgemäß wird die von Nukleinsäuren befreite Fraktion zunächst einer Ionenaustauscher-Chromatographie (Kationenaustauscher), z.B. mittels einer Poros HS 50-Säule, unterzogen. Anschließend wird, sofern ein als Reinigungshilfe dienendes Protein-tag, wie z.B. ein (Poly)His-, ein Biotinylierungspeptid, Streptacin® oder ein Maltose-bindendes Protein, mit dem TdT-Derivat verbunden ist, d.h. mitexprimiert wurde, eine Affinitätschromatographie angeschlossen. Im Fall von mit einem (Poly)His-Peptid verbundenen TdT-Expressionsprodukt sind insbesondere die kommerziell angebotenen Nickel-Chelat-Säulen für diesen Reinigungsschritt geeignet. Die erhaltene TdT-Fraktion wird anschließend durch eine geeignete hydrophobe Chromatographie, wie beispielsweise an Phenyl-Sepharose fast flow (ff), weiter gereinigt. Die beschriebene Reinigungsmethode liefert eine hochreine Terminale Transferase, die frei von kontaminierenden Enzymaktivitäten ist. In Abbildung 1 ist beispielhaft die Reinheit der Δ138-TdT mit einer Molmasse von 45,3 kDa im SDS-Gel gezeigt.

Die erfindungsgemäßen verkürzten rekombinanten Terminale Transferase-Peptide weisen überraschenderweise eine wesentlich höhere enzymatische Aktivität im Aktivitätstest in Cobalt-(Co²⁺-)Ionen haltigen Lösungen (sogen. "Co-System") als native Terminale Transferase-Derivate auf. Die native Terminale Transferase zeigt im Co-System eine ca. drei- bis vierfach höhere enzymatische Aktivität als im Zn/Mg-System. Im Gegensatz dazu zeigen die rekombinanten N-terminal verkürzten TdT-Derivate eine um das 20- bis 30-fach gesteigerte Aktivität. Das Δ138-TdT-Derivat zeigt z.B. im Co-System eine ca. 23-fach höhere Aktivität als im Zn/Mg-System. Im Vergleich zur nativen Terminalen Transferase weist die rekombinante Δ138-TdT somit eine bedeutend höhere Enzymaktivität im Co-System auf.

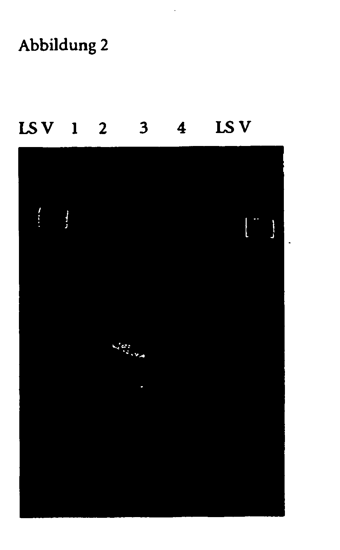

Darüber hinaus ist als überraschend anzusehen, dass das rekombinante Δ138-TdT-Derivat im Funktionstest eine deutlich bessere Performance als die native Terminale Transferase zeigt. Als Template für die Tailing-Reaktion wurde ein 30mer Oligonukleotid (5'-pTTG GGT AAC GCC AGG GTT TTC CCA GTC ACG OH-3') verwendet. Nach erfolgter Reaktion wurden die Reaktionsprodukte des Tailing-Experimentes auf einem 6 %-igen Agarose-Gel aufgetrennt und bewertet (Abb. 2). Dabei zeigte sich für die rekombinante TdT ein längeres und somit besseres Produkt der Tailing-Reaktion.

### Legenden zu den Abbildungen:

- Abbildung 1:: SDS-Gelelektrophorese der gereinigten Terminalen Transferase (Spur 1: Molekulargewichtsmarker Marke 12 (Fa. Novex); Spur 2: Terminale Transferase, 10 Units (Zn/Mg-System)
- Abbildung 2:: Oligo Tailing Reaktion (Spuren 1, 4: Oligonukleotid; Spur 2: Produkt der Tailing-Reaktion, 10 Units TdT, nativ; Spur 3: Produkt der Tailing-Reaktion, 10 Units TdT, rekombinant; LSV: DNA Molecular weight Marker V (pBR 322 DNA gespalten mit Hae III, 22 Fragmente 8-587 bp; Roche Diagnostics GmbH, Kat. Nr. 821 705))

### Legenden zu den Sequenzprotokollen:

- SEO ID NO.: 1: cDNA-Sequenz der TdT aus Kalbsthymus (Pos. 22-1581).
- SEO ID NO.: 2: Aminosäuresequenz der TdT aus Kalbsthymus (520 AS).
- SEO ID NO.: 3: 5'-Primer (58 N.).
- SEO ID NO.: 4: 5'-Primer (63 N.).
- SEO ID NO.: 5: 5'-Primer (60 N.).
- SEQ ID NO.: 6: 3'-Primer (42 N.).
- SECZID NO.: 7: Nukleinsäuresequenz kodierend für die verkürzte TdT, Δ 138-TdT, mit His-tag (1187 N.).
- SEO ID NO.: 8: Aminosäuresequenz der verkürzten TdT, Δ 138-TdT, mit His-tag (392 AS).
- SEQ ID NO.: 9: Nukleinsäuresequenz kodierend für die verkürzte TdT, Δ 152-TdT, mit His-tag (1148 N.).
- SEO ID NO.: 10: Aminosäuresequenz der verkürzten TdT, Δ 152-TdT, mit His-tag (379 AS).
- SEO ID NO.: 11: Nukleinsäuresequenz kodierend für die verkürzte TdT, Δ 161-TdT, mit His-tag (1121 N.).
- SEO ID NO.: 12: Aminosäuresequenz der verkürzten TdT, Δ 161-TdT, mit His-tag (370 AS).

Die folgenden Beispiele erläutern die Erfindung weiter:

### Rekombinante DNA-Technik

Zur Manipulation von DNA wurden Standardrnethoden benutzt, wie sie bei Sambrook, J. et al. (1989) Molecular cloning: A laboratory manual. Cold Spring Harbor Laboratory Press, Cold Spring Harbor, New York beschrieben sind. Bei Verwendung von Kits wurde jeweils nach den Empfehlungen der Hersteller vorgegangen. Die verwendeten molekularbiologischen Reagenzien wurden nach den Angaben des Herstellers eingesetzt.

### Beispiel 1:

### Erzeugung der verkürzten TdT-Gene:

Ausgehend von einem cDNA-Klon (Kowai et al. (1986), *Nucleic Acids Res.* 14: 5777-5792) mit einem Insert von 1923 bp gemäß SEQ ID NO.: 1, das den kompletten Leserahmen des Gens, das für die Terminale Transferase gemäß SEQ ID NO.: 2 aus Kalbsthymus kodiert, enthält, wurden Oligonukleotide gemäß SEQ ID 3 - 6 entworfen, die die Isolierung verkürzter Gene aus dem kompletten Leserahmen mittels PCR ermöglichen. Dabei wurden die 5'-primer (SEQ ID No.: 3-5) jeweils so entworfen, dass sie stromabwärts des kodierenden Bereiches die Codons für die Aminosäuren Met-Arg-Gly-Ser-His-His-His-His-His-His enthalten, so dass die verkürzten TdT-Peptide N-terminal mit einem His-tag fusioniert werden. Die 5'-primer enthalten keine Erkennungssequenz für eine Restriktionsendonuklease, sondern sollten am 5'-Ende über ein stumpfes Ende kloniert werden, während der 3'-primer downstream der kodierenden Region die Erkennungssequenz für die Restriktionsendonuklease *Hind*III besitzt.

Zur Isolierung eines Genes, das für eine um N-terminal 138 Aminosäuren verkürzte TdT (Δ138-TdT) kodiert, wurde die PCR-Reaktion mit den Oligonukleotiden gemäß SEQ ID NO.: 3 (5'-primer) und SEQ ID No.: 6 (3'-primer) durchgeführt. Das gewonnene PCR-Produkt gemäß SEQ ID No.: 7, das für die Δ138-TdT mit His-tag gemäß SEQ ID No.: 8 kodiert, wurde mittels Sequenzierung überprüft.

Zur Isolierung eines Genes, das für eine um N-terminal 152 Aminosäuren verkürzte TdT (Δ152-TdT) kodiert wurde die PCR-Reaktion mit den Oligonukleotiden gemäß SEQ ID NO.: 4 (5'-primer) und SEQ ID No.: 6 (3'-primer) durchgeführt. Das gewonnene PCR-Produkt gemäß SEQ ID No.: 9, das für die Δ152-TdT mit His-tag gemäß SEQ ID No.: 10 kodiert, wurde mittels Sequenzierung überprüft.

Zur Isolierung eines Genes, das für eine um N-terminal 161 Aminosäuren verkürzte TdT (Δ161-TdT) kodiert wurde die PCR-Reaktion mit den Oligonukleotiden gemäß SEQ ID NO.: 5 (5'-primer) und SEQ ID No.: 6 (3'-primer) durchgeführt. Das gewonnene PCR-Produkt gemäß SEQ ID No.: 11, das für die Δ161-TdT mit His-tag gemäß SEQ ID No.: 12 kodiert, wurde mittels Sequenzierung überprüft.

### Konstruktion der Expressionsplasmide der verkürzten TdT-Peptide

Zur Expression der TdT wurden die verkürzten Gene jeweils in Expressionsvektoren so kloniert, dass die Strukturgene jeweils in der richtigen Orientierung unter Kontrolle eines geeigneten Promotors, bevorzugt eines IPTG induzierbaren Promotors wie z.B. *lac-, lac*UV5-, *tac-* oder T5-Promotor, besonders bevorzugt des lac-Promotors insertiert ist. Dazu wurde das jeweilige PCR-Produkt mittels *Hind*III am 3'-Ende nachgeschnitten während das 5'-Ende nicht verändert wurde, die Restriktionsansätze durch Agarosegelelektrophorese aufgetrennt und das 1181 Bp große Fragment für Δ138-TdT-Gen, das 1139 bp große Fragment für das Δ152-TdT-Gen und das 1115 bp große Fragment für das Δ161-TdT-Gen, jeweils mit einer Nukleinsäure, die für den His-tag kodiert, aus dem Agarosegel isoliert. Zur Expression wurden verschiedene Expressionsplasmide wie z.B. pUC, pDS, pQE, pKK, bevorzugt aber pUC18 (Yanisch-Perron et al. (1985) *Gene* 33: 103-119) eingesetzt. pUC18 wurde für die Insertion der Gene für die verkürzten TdT-Peptide zunächst mit *Eco*RI (Roche Diagnostics) nach Herstellerangaben geschnitten, durch Inkubation bei 65 °C für 15 min wurde die Restriktionsendonuklease *Eco*RI inaktiviert und das entstandene überhängende Ende mit Klenow-Polymerase (Roche Diagnostics) nach Herstellerangaben zu einem stumpfen Ende aufgefüllt. Durch erneute Inkubation bei 65 °C für 15 min die Klenow-Polymerase inaktiviert. Anschließend wurde das Vektorfragment mit HindIII (Roche Diagnostics) geschnitten, der Restriktionsansatz durch Agarosegelelektrophorese aufgetrennt und das resultierende Vektorfragment von ca. 2656 bp aus dem Agarosegel isoliert. Das so gewonnene Vektorfragment wurde getrennt und mit den isolierten PCR-Produkten für die verkürzten TdT-Peptide ligiert. Die ordnungsgemäße Insertion der Gene wurde mittels Restriktionskontrolle und Sequenzierung überprüft. Die so entstandenen Plasmide pUC18Δ138-TdT, pUC18Δ152-TdT und pUC18Δ161-TdT (s. Abb. 1) wurden getrennt zur Expressionskontrolle in verschiedene *E. coli*-Stämme zusammen mit dem Helferplasmid pUBS520 cotransformiert. Das Helferplasmid pUBS520 (Brinkmann *et al.,* 1989, *Gene* **85**: 109-114) trägt u.a. das *lacI*^{*q*}-Gen, das für den Lac-Repressor kodiert und das *dna*Y-Gen, das für die in *E. coli* seltene tRNA^{ARG} (erkennt die Codons AGA und AGG) kodiert (Garcia *et al.,* 1986, *Cell* **45**: 453-459). Als Selektionsmarker wird das Kanamycin-Resistenzgen aus dem Transposon TN903 verwendet.

### Beispiel 2:

### Transformation der Expressionsplasmide pUC18Δ138-TdT, pUC18Δ152-TdT und pUC18Δ161-TdT in verschiedene E. coli-Expressionsstämme

Kompetente Zellen verschiedener *E. coli*-Stämme wurden entsprechend der Methode nach Hanahan (*J. Mol. Biol.* 1983, Vol. 166: 557) hergestellt. 200 µl derart hergestellter Zellen wurden mit 20 ng isolierter Expressionsplasmid-DNA pUC18Δ138-TdT, pUC18Δ152-TdT bzw. pUC18Δ161-TdT und 40 ng Helferplasmid-DNA versetzt. Nach 30 min. Inkubation auf Eis erfolgte ein Hitzeschock (90 sec bei 42 °C). Anschließend wurden die Zellen in 1 ml LB-Medium überführt und zur phänotypischen Expression 1 Stunde bei 37 °C in LB-Medium inkubiert. Aliquote dieses Transformationsansatzes wurden aufLB-Platten mit Ampicillin und Kanamycin als Selektionsmarker ausplattiert und 15 Stunden bei 37 °C inkubiert. Bevorzugte Stämme sind *E. coli* K12 C600, DH5α, LE392, JM83, JM105, NM522, M15, RR1Δ15, UT5600, TG1, A1200, oder die Stämme *E. coli* B, BL21, HB101, besonders bevorzugt *Escherichia coli* UT5600.

### Beispiel 3:

### Expression der verkürzten TdT-Gene in E. coli

Zur Expression des Gens, das für die verkürzten TdT-Peptide kodiert, wurden plasmidhaltige Klone in 3 ml LBₐₘₚₖₐₙ-Medium angeimpft und bei 37 °C im Schüttler inkubiert. Bei einer optischen Dichte von 0,5 (gemessen bei 550 nm, OD₅₅₀) wurden die Zellen mit 0,5 mM IPTG induziert und 4 h bei 37 °C im Schüttler inkubiert. Anschließend wurde die optische Dichte der einzelnen Expressionsklone bestimmt, ein Aliquot, das einer OD₅₅₀ₙₘ von 5.0/ml entspricht, entnommen und die Zellen abzentrifugiert (10 min, 6000 rpm, 4 °C). Das Zellpellet wurde in 400 µl TE-Puffer (50 mM TRIS/50 mM EDTA, pH 8,0) resuspendiert, die Zellen durch Ultraschall aufgeschlossen und durch Zentrifugation die lösliche Proteinfraktion von der unlöslichen Proteinfraktion getrennt (10 min, 13800 rpm, 4 °C). Alle Fraktionen wurden mit SDS- und 2-Mercaptoethanol-haltigen Auftragspuffer versetzt und die Proteine durch Kochen (5 min 100 °C) denaturiert. Anschließend wurden je 10 µl mittels eines analytisches SDS-Gels (10 %) analysiert (Laemmli U.K. 1970 *Nature* 227: 555-557).

Nach Auswertung des SDS-Gels ist eine klare Uberexpression der verkürzten TdT-Fragmente erkennbar. Bei ca. 45 kDa (Δ138-TdT) bzw. 44 kDa (Δ152-TdT) bzw. 43 kDa (Δ161-TdT) ist eine überexprimierte, zusätzliche Bande zu erkennen, die bei den nicht induzierten bzw. induzierten, aber nicht plasmidhaltigen Kontroll-Klonen nicht auftaucht. Auch bei den hohen Wachstumstemperaturen wurden bei Verwendung dieser Expressionsstrategie alle TdT-Fragmente in der löslichen Proteinfraktion detektiert, während keine entsprechende Bande auf gleicher Höhe in der unlöslichen Proteinfraktion zu erkennen ist.

### Beispiel 4:

### Bestimmung der Terminale Transferase-Aktivität

Zur Bestimmung der Terminalen Transferase-Aktivität wurden verschiedene Tests durchgeführt.

### 1. Nichtradioaktiver Test (Test A)

Während der Reinigung wurde die Terminale Transferase-Aktivität in den Fraktionen mittels eines nicht-radioaktiven Testsystems detektiert. Dazu wurde der "DIG Oligo 3'-End Labeling"-Kit (Roche Diagnostics GmbH, Kat.Nr. 1 362 372) verwendet. Die Inkubationszeit wurde dabei auf 30 Minuten verlängert.

### 2. Radioaktive Testsysteme

### A. Test im Zn/Mg-System (Test B)

Die Terminale Transferase-Aktivität der Pools wurde mittels eines radioaktiven Testsystems bestimmt, das Zink- und Magnesium-Ionen enthält. Der Test auf Terminale Transferase-Aktivität wurde dabei in 60 µl Testvolumen (40 mM Kaliumkakodylat, pH 6,8, 0,33 mM ZnSO₄, 10 mM MgCl₂, 1 mM dATP, 0.1 AB Poly d(pT)₆,12,5 pM [3H]-dATP) durchgeführt. Terminale Transferase (10 µl) wurde in geeigneten Verdünnungen zugegeben. Nach Inkubation für 30 min. bei 37 °C wurde die Reaktion mit 10 % TCA-Lösung (1000 µl) abgestoppt. Das gebildete, radioaktiv markierte Produkt wurde nach der Fällung auf Nitrocellulose-Filter gewaschen. Die Einbaurate an Radioaktivität wurde im Szintilationszähler gemessen und die Terminale Transferase-Aktivität der Probe berechnet. Eine Enzymeinheit wurde dabei als die Menge an Terminaler Transferase definiert, die in 60 min. bei 37 °C den Einbau von 1.0 nMol dAMP in säureunlösliches Produkt bewirkt.

Dieser Test wird zur routinemäßigen Aktivitätsbestimmung der nativen und rekombinanten Terminalen Transferase verwendet.

### B. Test im Co-System (Test C)

Die Terminale Transferase-Aktivität wurde des weiteren mit einem Testsystem, das Cobalt-Ionen enthält, bestimmt. Dieser Test wurde in 120 µl Testvolumen (200 mM Kaliumkakodylat, pH 7,2 , 1 mM CoCl₂, 1 mM dTTP, 0.1 AB Poly d(pT)₆, 37,5 pMol [3H]-dTTP) durchgeführt. Terminale Transferase (10 µl) wurde in geeigneten Verdünnungen zugegeben. Nach Inkubation für 30 min. bei 37 °C wurde die Reaktion mit 10 % TCA-Lösung (1000 µl) abgestoppt. Das gebildete, radioaktiv markierte Produkt wurde nach der Fällung auf Nitrocellulose-Filter gewaschen. Die Einbaurate an Radioaktivität wurde im Szintilations-Zähler gemessen und die Terminale Transferase-Aktivität der Probe berechnet. Eine Enzymeinheit wurde dabei als die Menge an Terminaler Transferase definiert, die in 60 min. bei 37 °C den Einbau von 1.0 nMol dTMP oder dATP in säureunlösliches Produkt unter Verwendung von d(pT)₆ als Primer bewirkt.

### Test auf kontaminierende Aktivitäten

Der Test auf das Vorhandensein von kontaminierenden Fremdaktivitäten wurde in einer Lösung bestehend aus 10 mM Tris/HCl, pH 7.5,10 mM MgCl₂, 1 mM DTE durchgeführt.

Geeignete Proben der einzelnen Enzymfraktionen wurden mit den entsprechenden Nukleinsäuren inkubiert. Sogenannte Nicking-Aktivität wurde durch Inkubation mit dem Plasmid pBR322 (1 µg) für 2-16 Stunden bei 37 °C nachgewiesen. Unspezifische Nukleasen wurden durch Inkubation mit Lambda-DNA/*Eco*RI, *Hind*III (1 µg) für 2-16 Stunden bei 37 °C nachgewiesen.

Für den Test auf Kontamination mit Exonukleasen wurden die Proben mit 4 µg [3H]-markierter DNA für 4 Stunden bei 37 °C inkubiert und danach die freigesetzten [3H]-markierten Nukleotide bestimmt.

### Beispiel 5:

### Reinigung der Terminalen Transferase

Als Ausgangsmaterial für die Reinigung der rekombinanten Terminaler Transferase wurden *E. coli* K12 UT5600-Zellen verwendet, die das Terminale Transferase-Gen überexprimieren (siehe oben).

Die Reinigung der TdT erfolgt bei 4 °C. Die Reinigung erfolgt nach Zellaufschluss und Abtrennung der Nukleinsäuren durch eine Folge von chromatographischen Schritten. Das Reinigungsverfahren liefert eine rekombinante TdT, die frei von kontaminierenden Enzymaktivitäten ist.

### Verwendete Lösungen:

Puffer A: 50 mM Tris/HCl, pH 7.6, 0.5 M NaCl, 50 mM LiCl
Puffer B: 50 mM KPO₄ , pH 6,0, 5% Glycerin.
Puffer C: 50 mM Tris/HCl, pH 7.6, 0.5 M NaCl, 5% Glycerin,
Puffer D: 20 mM KPO₄, pH 7.0, 1,3 M Ammoniumsulfat, 5% Glycerin.
Lagerpuffer: 60 mM KPO₄, pH 7,2,150 mM KCl, 1 mM 2-Mercaptoethanol,
   0,5 % Triton X-100, 50 % Glycerin.

### Aufschluss der Zellen:

Ca. 1100 g Zellen von *E. coli* K12 UT5600 wurden mit 4000 ml Puffer A versetzt, aufgetaut und suspendiert. Die Suspension wurde mit 20 ml 0,1 M PMSF Lösung (Roche Diagnostics GmbH, Kat. Nr. 2 36 608) versetzt. Anschließend erfolgte der Aufschluss der Zellen mittels Hochdruckdispersion (Gaulin Lab-60) unter Kühlung (Temperatur: <10°C). Der erreichte Aufschlussgrad der Zellsuspension betrug dabei typischerweise 40-50 %.

### Fällung von Nukleinsäuren:

Anschließend erfolgte eine Entfernung der Nukleinsäuren mittels Polyminfällung. 100 ml einer 10 %-igen Polymin-P-Lösung wurden tropfenweise zugegeben. Bei unvollständiger Fällung erfolgte eine weitere tropfenweise Zugabe. Nach Inkubation für 30 min bei 4 °C erfolgte eine Zentrifugation (30 min, 5000 upm, 4 °C).

### Chromatographische Reinigungen:

### Chromatographie an Poros HS 50-Säule:

Der dialysierte Zentrifugationsüberstand wurde auf eine mit Puffer B + 0,2 M NaCl äquilibrierte Poros HS 50 Säule (9 cm x 20 cm, PerSeptiv) aufgezogen und mit ca. 101 Puffer B +0,2 M NaCl gewaschen. Die Elution des Enzyms erfolgte mit einem linearen Gradienten aus Puffer B + 200 mM NaCl und Puffer B+1 M NaCl in einem Gesamtvolumen von 8 l. Die Fließgeschwindigkeit betrug 100 ml pro min, die Fraktionsgröße 100 ml. Die Terminale Transferase eluiert bei einer NaCl-Konzentration von 300 mM bis 700 mM.

### Affinitätschromatographie an Ni-Chelat-Säule:

Der klare Pool wurde mit K₂HPO₄ auf pH 7,5 eingestellt und mit 1/100 Puffer C +1 M Imidazole versetzt und anschließend auf eine mit Puffer C + 10 mM Imidazole äquilibrierte und mit Nickel beladene Chelating Sepharose ff-Säule (2,6 cm x 10 cm, Pharmacia) aufgezogen; danach wurde mit ca. 800 ml Puffer C+ 20 mM Imidazole waschen, danach mit Puffer C + 30 mM Imidazol gewaschen. Das Enzym wurde mit einem linearen Gradienten aus Puffer C + 30 mM Imidazol und Puffer C + 1 M Imidazole in einem Gesamtvolumen von 600 ml eluiert. Die Fließgeschwindigkeit betrug 12 ml pro Minute, die Fraktionsgröße 25 ml pro Fraktion. Das Enzym eluierte bei einer Konzentration von 50 mM bis 200 mM Imidazol. Alle aktiven Fraktionen wurden vereinigt. Dem Pool wurde festes Ammoniumsulfat bis zu einer Konzentration von 1,3 M zugegeben.

### Chromatographie an Phenyl Sepharose ff:

Der Pool wurde danach auf eine mit Puffer D äquilibrierte Phenyl-Sepharose ff-Säule (2,6 cm x 10 cm, Pharmacia) aufgezogen. Die Säule wurde zuerst mit ca. 400 ml Puffer D, danach mit ca. 600 ml Puffer D + 500 mM Ammoniumsulfat gewaschen. Die Elution des Enzyms erfolgte in diesen Waschschritt. Die Fließgeschwindigkeit betrug 10 ml pro min, die Fraktionsgröße war 10 ml.

Die aktiven Fraktionen wurden gepolt und gegen Lagerpuffer dialysiert. Zur Analytik der Reinheit wurde das gereinigte Protein mit SDS- und 2-Mercaptoethanol-haltigen Auftragspuffer versetzt und die Probe durch Kochen (5 min 100 °C) denaturiert. Anschließend wurde eine Probe (20 µl) mittels eines analytisches SDS-Gels (4-20 %) analysiert (Laemmli U.K. 1970 *Nature* 227: 555-557). Die beschriebene Reinigungsmethode liefert eine hochreine Terminale Transferase mit einer Molmasse von 45,3 kDa (Abb. 1).

### Beispiel 6:

### Vergleich der Aktivitäten der nativen und rekombinanten Terminalen Transferasen

Aufgrund der verbesserten Performance der rekombinanten Terminalen Transferase in der Tailing-Reaktion wurden die Enzymaktivitäten der beiden Terminalen Transferasen in zwei unterschiedlichen Testsystemen untersucht. Dazu wurden das Zn/Mg-System (Test B) und das Co-System (Test C) verwendet.

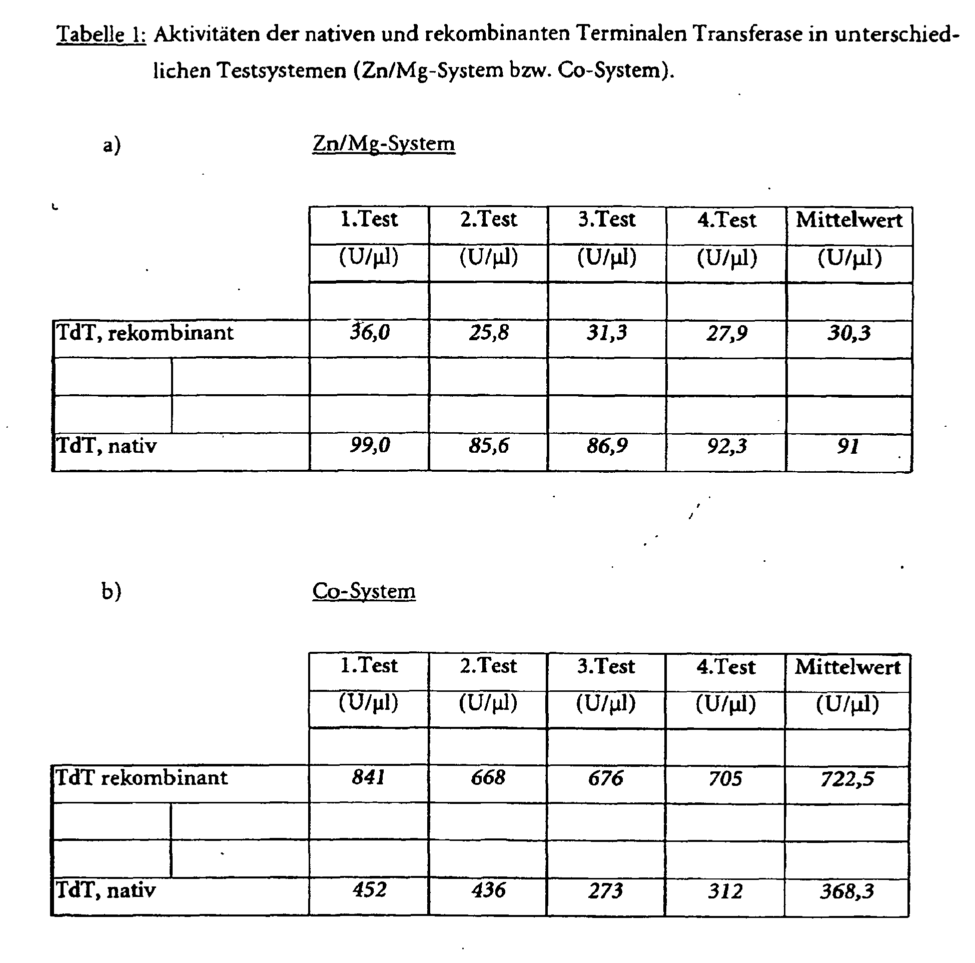

Die native Terminale Transferase zeigt im Co-System eine ca. drei- bis vierfach höhere Aktivität als im Zn/Mg-System. Im Gegensatz dazu zeigt die rekombinante Terminale Transferase im Co-System eine ca. 23-fach höhere Aktivität als im Zn/Mg-System. Im Vergleich zur nativen Terminaler Transferase zeigt die rekombinante Terminale Transferase somit eine stärkere Verbesserung der Enzymaktivität im Co-System.

Dieser Unterschied in der Aktivität könnte die verbesserte Performance der rekombinanten Terminalen Transferase in der Tailing-Reaktion erklären.

### Beispiel 7:

### Funktionstest für Terminale Transferase

Die erhaltene rekombinante Terminale Transferase wurde in einem Funktionstest untersucht. Der Funktionstest bestand aus einer Oligo Tailing-Reaktion. Dazu wurden jeweils 10 Einheiten der rekombinanten TdT und der nativen TdT in dem "Dig Oligonukleotid Tailing"-Kit (Kat. Nr. 1 417 231, Roche Diagnostics GmbH) eingesetzt. Als Template für die Tailing-Reaktion wurden 100 pmol eines 30 mer Oligo Nukleotids (5'-pTTG GGT AAC GCC AGG GTT TTC CCA GTC ACG OH-3') verwendet.

Die Reaktionsprodukte des Tailing-Experimentes wurden auf einem 6 %-igen Agarose-Gel aufgetrennt und bewertet (Abb. 2). Dabei zeigte sich für die rekombinante TdT ein längeres und somit besseres Produkt der Tailing-Reaktion.

### Vergleich der erfindungsgemäßen TdT mit bekannten Präparaten

**Tabelle 2: Aktivitäten der nativen und rekombinanten TdT sowie der TdT-Präparate verschiedener Hersteller in unterschiedlichen Testsystemen.**

| **Ansatz/Charge** | **Quelle** | **Zn/Mg-System [U/µl]** | **Co-System [U/µl]** |
|---|---|---|---|
| Verkürzte TdT | *E.coli,* rek. (Kalbsthymus) | 30,3 | 722,5 |
| TdT, nativ [55 U/µl] | Kalbsthymus | 91,0 | 368,3 |
| TdT, nativ Stratagene Lot: 0610233 [28 U/µl] | Kalbsthymus | 9,4 | 47,8 |
| TdT, nativ Amersham Pharmacia Lot: 5473 [15 U/µl] | Kalbsthymus | 11,3 | 72,3 |
| TdT BRL Lot: 1093333 [15 U/µl] | Baculovirus, rek. (Kalbsthymus) | 2,7 | 20,4 |
| TdT, nativ Promega Lot: 91884 [20 U/µl] | Kalbsthymus | 39,9 | 70,9 |
| TdT NEBL Lot: 2A [20 U/µl] | *E.coli,* rek. (Kalbsthymus) | 24,7 | 42,4 |

### Literatur:

Brinkmann U., Mattes R.E. und Buckel P. (1989),
   *Gene* **85**: pp. 109-114
Boulé J.-B., Johnson E., Rougeon F. und Papanicolaou C. (1998),
   *Molecular Biotechnology* **10**: pp. 199-208
Chang L.M., Plevani P. und Bollum F.J.. (1982),
   *J. Biol. Chem.* **257**(10): pp. 5700-5706
Chang L.M. und Bollum F.J. (1986),
   *CRC Crit Rev Biochem* **21**(1): pp. 27-52
Chang L.M., Rafter E., Rusquet-Valerius, Peterson R.C., White S.T. und Bollum F.J.. (1986),
   *J. Biol. Chem*. **263** (25): pp. 12509-12513
Deibel Jr. M.R. und Coleman M.S.(1979),
   *J. Biol. Chem.* **254**(17): pp 8634-8640
Garcia G.M., Mar P.K., Mullin D.A., Walker J.R. und Prather N.E (1986),
   *Cell* **45**: pp.453-459
Hanahan D. (1983),
   *J. Mol. Biol.* Vol. 166 pp. 557
Koiwai O., Yokota T., Kageyama T., Hirose T., Yoshida S. und Arai K.-I. (1986),
   *Nucleic Acid Research* **14** (14), pp. 5777-5792
Laemmli U.K. (1970),
   *Nature* **227:** pp. 555-557
Peterson R.C., Cheung L.C., Mattaliano R.J., White S.T., Chang L.M.S. und Bollum F.J. (1985),
   *J Biol Chem* **260** (19):pp10495-502
Sambrook J., Fritsch E.F. und Maniatis T., (1989),
   In *Molecular cloning. A Laboratory Manual* second Edition Cold Sprig Harbor Laboratory Press NY (USA)
Yang B., Gathy K.N. und Coleman M.S. (1995);
   *Nucleic Acids Research* **23** (11): pp. 2041-2048
US 5 037 756
   Inventors Bollum F.J., Chang L.M.S. und Peterson R.C.

## Patentansprüche

1. Verkürztes terminales Desoxynukleotidyltransferase (TdT)-Derivat, **dadurch gekennzeichnet, dass** das Derivat gegenüber der nativen TdT um 100 bis 160 Aminosäuren N-terminal verkürzt ist und eine um das 20- bis 30-fach höhere Enzymaktivität in Co²⁺-haltigen Lösungen als im Zn/Mg-System aufweist.

2. TdT-Derivat nach Anspruch 1, **dadurch gekennzeichnet, dass** das Derivat ein Molekulargewicht zwischen 36 und 46 kDa (SDS-Page) aufweist und aus Kalbsthymus erhältlich ist.

3. TdT-Derivat nach Anspruch 1 oder 2, **dadurch gekennzeichnet, dass** das Derivat um 138 Aminosäuren N-terminal verkürzt ist und ein Molekulargewicht von zwischen 44 und 46 kDa (SDS-Page) aufweist.

4. Verfahren zur rekombinanten Herstellung einer verkürzten TdT aus Kalbsthymus nach einem der Ansprüche 1 bis 3, **dadurch gekennzeichnet, dass** folgende Schritte durchgeführt werden:
a) Transformation einer Wirtszelle mit einem Expressionsplasmid enthaltend ein Nukleinsäurefragment, welches für ein N-terminal verkürztes TdT-Fragment kodiert und gegebenenfalls mit einem Nukleinsäurefragment fusioniert ist, das für ein die anschließende Reinigung vereinfachendes Protein-tag kodiert,
b) Kultivierung der Wirtszelle zur Expression der rekombinanten verkürzten TdT unter für die jeweilige Wirtszelle geeigneten Kultivierungsbedingungen,
c) Isolierung der rekombinanten verkürzten TdT aus der Wirtszelle und
d) Einsatz und Überprüfung der rekombinanten verkürzten TdT in Funktionsassays.

5. Verfahren nach Anspruch 4, **dadurch gekennzeichnet, dass** das Expressionsplasmid ein Nukleinsäurefragment gemäß SEQ ID NO.: 7, SEQ ID NO.: 9 oder SEQ ID NO.: 11 enthält.

6. Verfahren nach Anspruch 4, **dadurch gekennzeichnet, dass** das Nukleinsäurefragment für eine verkürzte Terminale Transferase gemäß SEQ ID NO.: 8, SEQ ID NO.: 10 oder SEQ ID NO.: 12 kodiert.

7. Verfahren nach einem der Ansprüche 4 oder 5, **dadurch gekennzeichnet, dass** das Expressionsplasmid ein Nukleinsäurefragment gemäß SEQ ID NO.: 7 enthält.

8. Verfahren nach einem der Ansprüche 4 oder 6, **dadurch gekennzeichnet, dass** die verkürzte Terminale Transferase die Aminosäuresequenz gemäß SEQ ID NO.: 8 enthält.

9. Verwendung des verkürzten TdT-Derivates gemäß einem der Ansprüche 1 bis 3 für Oligotailing-Anwendungen.

10. Verwendung des verkürzten TdT-Derivates gemäß einem der Ansprüche 1 bis 3 in einer Methode zur in vitro-Bestimmung von Zelltod (Apoptosis).

## Claims

1. Truncated terminal deoxynucleotidyl transferase (TdT) derivative, **characterized in that** in comparison to the native TdT the derivative is shortened at the N-terminus by 100 to 160 amino acids and has a 20- to 30-fold higher enzyme activity in solutions containing Co²⁺ ions than in the Zn/Mg system.

2. TdT derivative as claimed in claim 1, **characterized in that** the derivative has a molecular weight between 36 and 46 kDa (SDS page) and is obtainable from calf thymus.

3. TdT derivative as claimed in claim 1 or 2, **characterized in that** the derivative is shortened at the N-terminus by 138 amino acids and has a molecular weight between 44 and 46 kDa (SDS page).

4. Method for the recombinant production of a truncated TdT from calf thymus as claimed in claims 1 to 3 which is **characterized in that** the following steps are carried out:
a) transformation of a host cell with an expression plasmid containing a nucleic acid fragment which codes for an N-terminally truncated TdT fragment and is optionally fused with a nucleic acid which codes for a protein tag that facilitates the subsequent purification,
b) culture of the host cell to express the recombinant truncated TdT under suitable culture conditions for the respective host cell,
c) isolation of the recombinant truncated TdT from the host cell and
d) use and examination of the recombinant truncated TdT in a functional assay.

5. Method as claimed in claim 4, **characterized in that** the expression plasmid contains a nucleic acid fragment according to SEQ ID NO.: 7, SEQ ID NO.: 9 or SEQ ID NO.: 11.

6. Method as claimed in claim 4, **characterized in that** the nucleic acid fragment codes for a truncated terminal transferase according to SEQ ID NO.: 8, SEQ ID NO.: 10 or SEQ ID NO.: 12.

7. Method as claimed in one of the claims 4 or 5, **characterized in that** the expression plasmid contains a nucleic acid fragment according to SEQ ID NO.: 7.

8. Method as claimed in one of the claims 4 to 6, **characterized in that** the truncated terminal transferase contains the amino acid sequence according to SEQ ID NO.: 8.

9. Use of the truncated TdT derivative as claimed in one of the claims 1 to 3 for oligotailing applications.

10. Use of the truncated TdT derivative as claimed in one of the claims 1 to 3 in a method for determining cell death (apoptosis).

## Revendications

1. Dérivé de désoxynucléotidyltransférase terminale (TdT) raccourcie, **caractérisé en ce que** par rapport à la TdT naturelle, le dérivé est raccourci de 100 à 160 acides aminés à sa terminaison N et présente, dans des solutions contenant du Co²⁺, une activité enzymatique de 20 à 30 fois supérieure à celle qu'il présente dans des systèmes Zn/Mg.

2. Dérivé de TdT selon la revendication 1, **caractérisé en ce que** le dérivé présente un poids moléculaire compris entre 36 et 46 kDa (SDS-Page) et qu'il peut être obtenu à partir de thymus de veau.

3. Dérivé de TdT selon la revendication 1 ou 2, **caractérisé en ce que** le dérivé est raccourci de 138 acides aminés à sa terminaison N et présente un poids moléculaire compris entre 44 et 46 kDa (SDS-Page).

4. Procédé de préparation recombinante d'une TdT raccourcie, à partir de thymus de veau, selon l'une quelconque des revendications 1 à 3, **caractérisé en ce que** l'on exécute les étapes suivantes :
a) transformation d'une cellule hôte à l'aide d'un plasmide d'expression comprenant un fragment d'acide nucléique qui code pour un fragment de TdT raccourci à sa terminaison N et qui est éventuellement fusionné avec un fragment d'acide nucléique qui code pour un marqueur protéinique facilitant la purification subséquente,
b) culture de la cellule hôte pour l'expression de la TdT raccourcie recombinante dans des conditions de culture appropriées pour la cellule hôte donnée,
c) isolement de la TdT raccourcie recombinante à partir de la cellule hôte, et
d) mise en oeuvre et test de l'activité de la TdT raccourcie recombinante dans des essais fonctionnels.

5. Procédé selon la revendication 4, **caractérisé en ce que** le plasmide d'expression contient un fragment d'acide nucléique correspondant aux séquences SEQ ID n°7, SEQ ID n° 9 ou SEQ ID n° 11.

6. Procédé selon la revendication 4, **caractérisé en ce que** le fragment d'acide nucléique code pour une transférase terminale raccourcie correspondant aux séquences SEQ ID n°8, SEQ ID n° 10 ou SEQ ID n° 12.

7. Procédé selon l'une quelconque des revendications 4 ou 5, **caractérisé en ce que** le plasmide d'expression contient un fragment d'acide nucléique correspondant à la séquence SEQ ID n° 7.

8. Procédé selon l'une quelconque des revendications 4 ou 6, **caractérisé en ce que** la transférase terminale raccourcie contient la séquence d'acides aminés correspondant à la séquence SEQ ID n°8.

9. Utilisation du dérivé de TdT raccourci selon l'une quelconque des revendications 1 à 3 pour des applications d'oligotailing.

10. Utilisation du dérivé de TdT raccourci selon l'une quelconque des revendications 1 à 3 dans une méthode destinée à la détermination in vitro de la mort cellulaire (apoptose).


### SEQUENCE LISTING

<110> Roche Diagnostics GmbH
<120> Rekombinante terminal Deoxynukleotidyltransferase mit verbesserter Funktionalität
<130> 21236
<160> 12
<170> PatentIn version 3.1
<210> 1
   <211> 1923
   <212> DNA
   <213> Calf thymus
<400> 1
   ctcttctgga gataccactt gatggcacag cagaggcagc atcagcgtct tcccatggat 60
   ccgctgtgca cagcctcctc aggccctcgg aagaagagac ccaggcaggt gggtgcctca 120
   atggcctccc ctcctcatga catcaagttt caaaatttgg tcctcttcat tttggagaag 180
   aaaatgggaa ccacccgcag aaacttcctc atggagctgg ctcgaaggaa aggtttcagg 240
   gttgaaaatg agctcagtga ttctgtcacc cacattgtag cagaaaacaa ctctggttca 300
   gaggttctcg agtggcttca ggtacagaac ataagagcca gctcgcagct agaactcctt 360
   gatgtctcct ggctgatcga aagtatggga gcaggaaaac cagtggagat tacaggaaaa 420
   caccagcttg ttgtgagaac agactattca gctaccccaa acccaggctt ccagaagact 480
   ccaccacttg ctgtaaaaaa gatctcccag tacgcgtgtc aaagaaaaac cactttgaac 540
   aactataacc acatattcac ggatgccttt gagatactgg ctgaaaattc tgagtttaaa 600
   gaaaatgaag tctcttatgt gacatttatg agagcagctt ctgtacttaa atctctgcca   660
   ttcacaatca tcagtatgaa ggatacagaa ggaattccct gcctggggga caaggtgaag   720
   tgtatcatag aggaaattat tgaagatgga gaaagttctg aagttaaagc tgtgttaaat 780
   gatgaacgat atcagtcctt caaactcttt acttctgttt ttggagtggg actgaagaca 840
   tctgagaaat ggttcaggat ggggttcaga tctctgagta aaataatgtc agacaaaacc 900
   ctgaaattca caaaaatgca gaaagcagga tttctctatt atgaagacct tgtcagctgc 960
   gtgaccaggg ccgaagcaga ggcggttggc gtgctggtta aagaggctgt gtgggcattt 1020
   ctgccggatg cctttgtcac catgacagga ggattccgca ggggtaagaa gattgggcat 1080
   gatgtagatt ttttaattac cagcccagga tcagcagagg atgaagagca acttttgcct 1140
   aaagtgataa acttatggga aaaaaaggga ttacttttat attatgacct tgtggagtca 1200
   acatttgaaa agttcaagtt gccaagcagg caggtggata ctttagatca ttttcaaaaa 1260
   tgctttctga ttttaaaatt gcaccatcag agagtagaca gtagcaagtc caaccagcag 1320
   gaaggaaaga cctggaaggc catccgtgtg gacctggtta tgtgccccta cgagaaccgt 1380
   gcctttgccc tgctaggctg gactggctcc cggcagtttg agagagacat ccggcgctat 1440
   gccacacacg agcggaagat gatgctggat aaccacgctt tatatgacaa gaccaagagg 1500
   gtatttctca aagcggaaag tgaagaagaa atctttgcac atctgggatt ggactacatt 1560
   gaaccatggg aaagaaatgc ttaggagaaa gctgtcaact tttttctttt ctgttctttt 1620
   tttcaggtta gacaaattat gcttcatatt ataatgaaag atgccttagt caagtttggg 1680
   attctttaca ttttaccaag atgtagattg cttctagaaa taagtagttt tggaaacgtg 1740
   atcaggcacc ccctgggtta tgctctggca agccatttgc aggactgatg tgtagaactc 1800
   gcaatgcatt ttccatagaa acagtgttgg aattggtggc tcatttccag ggaagttcat 1860
   caaagcccac tttgcccaca gtgtagctga aatactgtat acttgccaat aaaaatagga 1920
   aac 1923
<210> 2
   <211> 520
   <212> PRT
   <213> Calf thymus
<400> 2
   Met Ala Gln Gln Arg Gln His Gln Arg Leu Pro Met Asp Pro Leu Cys
   1               5                   10                  15
   Thr Ala Ser Ser Gly Pro Arg Lys Lys Arg Pro Arg Gln Val Gly Ala
               20                  25                  30
   Ser Met Ala Ser Pro Pro His Asp Ile Lys Phe Gln Asn Leu Val Leu
           35                  40                  45
   Phe Ile Leu Glu Lys Lys Met Gly Thr Thr Arg Arg Asn Phe Leu Met
       50                  55                  60
   Glu Leu Ala Arg Arg Lys Gly Phe Arg Val Glu Asn Glu Leu Ser Asp
   65                  70                  75                  80
   Ser Val Thr His Ile Val Ala Glu Asn Asn Ser Gly Ser Glu Val Leu
                   85                  90                  95
   Glu Trp Leu Gln Val Gln Asn Ile Arg Ala Ser Ser Gln Leu Glu Leu
               100                 105                 110
   Leu Asp Val Ser Trp Leu Ile Glu Ser Met Gly Ala Gly Lys Pro Val
           115                 120                 125
   Glu Ile Thr Gly Lys His Gln Leu Val Val Arg Thr Asp Tyr Ser Ala
       130                 135                 140
   Thr Pro Asn Pro Gly Phe Gln Lys Thr Pro Pro Leu Ala Val Lys Lys
   145                 150                 155                 160
   Ile Ser Gln Tyr Ala Cys Gln Arg Lys Thr Thr Leu Asn Asn Tyr Asn
                   165                 170                 175
   His Ile Asp Ala Phe Glu Ile Leu Ala Glu Asn Ser Glu Phe Lys Glu
               180                 185                 190
   Asn Glu Val Ser Tyr Val Thr Phe Met Arg Ala Ala Ser Val Leu Lys
           195                 200                 205
   Ser Leu Pro Phe Thr Ile Ile Ser Met Lys Asp Thr Phe Thr Glu Gly
       210                 215                 220
   Ile Pro Cys Leu Gly Asp Lys Val Lys Cys Ile Ile Glu Glu Ile Ile
   225                 230                 235                 240
   Glu Asp Gly Glu Ser Ser Glu Val Lys Ala Val Leu Asn Asp Glu Arg
                   245                 250                 255
   Tyr Gln Ser Phe Lys Leu Ser Val Phe Gly Val Gly Leu Lys Thr Ser
               260                 265                 270
   Glu Lys Trp Phe Arg Met Gly Phe Thr Phe Arg Ser Leu Ser Lys Ile
           275                 280                 285
   Met Ser Asp Lys Thr Leu Lys Lys Met Gln Lys Ala Gly Phe Leu Tyr
       290                 295                 300
   Tyr Glu Asp Leu Val Ser Cys Val Thr Arg Ala Glu Ala Glu Ala Val
   305                 310                 315                 320
   Gly Val Leu Val Lys Glu Ala Val Trp Ala Phe Leu Pro Asp Ala Phe
                   325                 330                 335
   Val Thr Met Thr Gly Gly Phe Arg Arg Gly Lys Lys Ile Gly His Asp
               340                 345                 350
   Val Asp Phe Leu Ile Thr Ser Pro Gly Ser Ala Glu Asp Glu Glu Gln
           355                 360                 365
   Leu Leu Pro Lys Val Ile Asn Leu Trp Glu Lys Lys Gly Leu Leu Leu
       370                 375                 380
   Tyr Tyr Asp Leu Val Glu Ser Thr Phe Glu Lys Phe Lys Phe Thr Leu
   385                 390                 395                 400
   Pro Ser Arg Gln Val Asp Thr Leu Asp His Phe Gln Lys Cys Phe Leu
                   405                 410                 415
   Ile Leu Lys Leu His His Gln Arg Val Asp Ser Ser Lys Ser Asn Gln
               420                 425                 430
   Gln Glu Gly Lys Thr Trp Lys Ala Ile Arg Val Asp Leu Val Met Cys
           435                 440                 445
   Pro Tyr Glu Asn Arg Ala Phe Ala Leu Leu Gly Trp Thr Gly Ser Arg
       450                 455                 460
   Gln Phe Glu Arg Asp Ile Arg Arg Tyr Ala Thr His Glu Arg Lys Met
   465                 470                 475                     480
   Met Leu Asp Asn His Ala Leu Tyr Asp Lys Thr Lys Arg Val Phe Leu
                   485                 490                 495
   Lys Ala Glu Ser Glu Glu Glu Ile Phe Ala His Leu Gly Leu Asp Tyr
               500                 505                 510
   Ile Glu Pro Trp Glu Arg Asn Ala
           515                 520
<210> 3
   <211> 58
   <212> DNA
   <213> Artificial Sequence, Primer
<400> 3
   atgagaggat cgcatcacca tcaccatcac agaacagact attcagctac cccaaacc        58
<210> 4
   <211> 63
   <212> DNA
   <213> Artificial Sequence, Primer
<400> 4
   atgagaggat cgcatcacca tcaccatcac aagactccac cacttgctgt aaaaaagatc      60
   tcc                                                                     63
<210> 5
   <211> 60
   <212> DNA
   <213> Artificial Sequence, Primer
<400> 5
   atgagaggat cgcatcacca tcaccatcac atctcccagt acgcgtgtca aagaaaaacc      60
<210> 6
   <211> 42
   <212> DNA
   <213> Artificial Sequence, Primer
<400> 6
   gcgcaagctt aagcatttct ttcccatggt tcaatgtagt cc 42
<210> 7
   <211> 1187
   <212> DNA
   <213> Calf thymus
<400> 7
   atgagaggat cgcatcacca tcaccatcac agaacagact attcagctac cccaaaccca 60
   ggcttccaga agactccacc acttgctgta aaaaagatct cccagtacgc gtgtcaaaga 120
   aaaaccactt tgaacaacta taaccacata ttcacggatg cctttgagat actggctgaa 180
   aattctgagt ttaaagaaaa tgaagtctct tatgtgacat ttatgagagc agcttctgta 240
   cttaaatctc tgccattcac aatcatcagt atgaaggata cagaaggaat tccctgcctg 300
   ggggacaagg tgaagtgtat catagaggaa attattgaag atggagaaag ttctgaagtt 360
   aaagctgtgt taaatgatga acgatatcag tccttcaaac tctttacttc tgtttttgga 420
   gtgggactga agacatctga gaaatggttc aggatggggt tcagatctct gagtaaaata 480
   atgtcagaca aaaccctgaa attcacaaaa atgcagaaag caggatttct ctattatgaa 540
   gaccttgtca gctgcgtgac cagggccgaa gcagaggcgg ttggcgtgct ggttaaagag 600
   gctgtgtggg catttctgcc ggatgccttt gtcaccatga caggaggatt ccgcaggggt 660
   aagaagattg ggcatgatgt agatttttta attaccagcc caggatcagc agaggatgaa 720
   gagcaacttt tgcctaaagt gataaactta tgggaaaaaa agggattact tttatattat 780
   gaccttgtgg agtcaacatt tgaaaagttc aagttgccaa gcaggcaggt ggatacttta 840
   gatcattttc aaaaatgctt tctgatttta aaattgcacc atcagagagt agacagtagc 900
   aagtccaacc agcaggaagg aaagacctgg aaggccatcc gtgtggacct ggttatgtgc 960
   ccctacgaga accgtgcctt tgccctgcta ggctggactg gctcccggca gtttgagaga 1020
   gacatccggc gctatgccac acacgagcgg aagatgatgc tggataacca cgctttatat 1080
   gacaagacca agagggtatt tctcaaagcg gaaagtgaag aagaaatctt tgcacatctg 1140
   ggattggact acattgaacc atgggaaaga aatgcttaag cttgcgc 1187
<210> 8
   <211> 392
   <212> PRT
   <213> Calf thymus
<400> 8
   Met Arg Gly Ser His His His His His His Arg Thr Asp Tyr Ser Ala
   1               5                   10                  15
   Thr Pro Asn Pro Gly Phe Gln Lys Thr Pro Pro Leu Ala Val Lys Lys
               20                  25                  30
   Ile Ser Gln Tyr Ala Cys Gln Arg Lys Thr Thr Leu Asn Asn Tyr Asn
           35                  40                  45
   His Ile Asp Ala Phe Glu Ile Leu Ala Glu Asn Ser Glu Phe Lys Glu
       50                  55                  60
   Asn Glu Val Ser Tyr Val Thr Phe Met Arg Ala Ala Ser Val Leu Lys
   65                  70                  75                  80
   Ser Leu Pro Phe Thr Ile Ile Ser Met Lys Asp Thr Phe Thr Glu Gly
                   85                  90                  95
   Ile Pro Cys Leu Gly Asp Lys Val Lys Cys Ile Ile Glu Glu Ile Ile
               100                 105                 110
   Glu Asp Gly Glu Ser Ser Glu Val Lys Ala Val Leu Asn Asp Glu Arg
           115                 120                 125
   Tyr Gln Ser Phe Lys Leu Ser Val Phe Gly Val Gly Leu Lys Thr Ser
       130                 135                 140
   Glu Lys Trp Phe Arg Met Gly Phe Thr Phe Arg Ser Leu Ser Lys Ile
   145                 150                 155                 160
   Met Ser Asp Lys Thr Leu Lys Lys Met Gln Lys Ala Gly Phe Leu Tyr
                   165                 170                 175
   Tyr Glu Asp Leu Val Ser Cys Val Thr Arg Ala Glu Ala Glu Ala Val
               180                 185                 190
   Gly Val Leu Val Lys Glu Ala Val Trp Ala Phe Leu Pro Asp Ala Phe
           195                 200                 205
   Val Thr Met Thr Gly Gly Phe Arg Arg Gly Lys Lys Ile Gly His Asp
       210                 215                 220
   Val Asp Phe Leu Ile Thr Ser Pro Gly Ser Ala Glu Asp Glu Glu Gln
   225                 230                 235                 240
   Leu Leu Pro Lys Val Ile Asn Leu Trp Glu Lys Lys Gly Leu Leu Leu
                   245                 250                 255
   Tyr Tyr Asp Leu Val Glu Ser Thr Phe Glu Lys Phe Lys Phe Thr Leu
               260                 265                 270
   Pro Ser Arg Gln Val Asp Thr Leu Asp His Phe Gln Lys Cys Phe Leu
           275                 280                 285
   Ile Leu Lys Leu His His Gln Arg Val Asp Ser Ser Lys Ser Asn Gln
       290                 295                 300
   Gln Glu Gly Lys Thr Trp Lys Ala Ile Arg Val Asp Leu Val Met Cys
   305                 310                 315                 320
   Pro Tyr Glu Asn Arg Ala Phe Ala Leu Leu Gly Trp Thr Gly Ser Arg
                   325                 330                 335
   Gln Phe Glu Arg Asp Ile Arg Arg Tyr Ala Thr His Glu Arg Lys Met
               340                 345                 350
   Met Leu Asp Asn His Ala Leu Tyr Asp Lys Thr Lys Arg Val Phe Leu
   355 360 365
   Lys Ala Glu Ser Glu Glu Glu Ile Phe Ala His Leu Gly Leu Asp Tyr
   370 375 380
   Ile Glu Pro Trp Glu Arg Asn Ala
   385 390
<210> 9
   <211> 1148
   <212> DNA
   <213> Calf thymus
<400> 9
   atgagaggat cgcatcacca tcaccatcac aagactccac cacttgctgt aaaaaagatc 60
   tcccagtacg cgtgtcaaag aaaaaccact ttgaacaact ataaccacat attcacggat 120
   gcctttgaga tactggctga aaattctgag tttaaagaaa atgaagtctc ttatgtgaca 180
   tttatgagag cagcttctgt acttaaatct ctgccattca caatcatcag tatgaaggat 240
   acagaaggaa ttccctgcct gggggacaag gtgaagtgta tcatagagga aattattgaa 300
   gatggagaaa gttctgaagt taaagctgtg ttaaatgatg aacgatatca gtccttcaaa 360
   ctctttactt ctgtttttgg agtgggactg aagacatctg agaaatggtt caggatgggg 420
   ttcagatctc tgagtaaaat aatgtcagac aaaaccctga aattcacaaa aatgcagaaa 480
   gcaggatttc tctattatga agaccttgtc agctgcgtga ccagggccga agcagaggcg 540
   gttggcgtgc tggttaaaga ggctgtgtgg gcatttctgc cggatgcctt tgtcaccatg 600
   acaggaggat tccgcagggg taagaagatt gggcatgatg tagatttttt aattaccagc 660
   ccaggatcag cagaggatga agagcaactt ttgcctaaag tgataaactt atgggaaaaa 720
   aagggattac ttttatatta tgaccttgtg gagtcaacat ttgaaaagtt caagttgcca 780
   agcaggcagg tggatacttt agatcatttt caaaaatgct ttctgatttt aaaattgcac 840
   catcagagag tagacagtag caagtccaac cagcaggaag gaaagacctg gaaggccatc 900
   cgtgtggacc tggttatgtg cccctacgag aaccgtgcct ttgccctgct aggctggact 960
   ggctcccggc agtttgagag agacatccgg cgctatgcca cacacgagcg gaagatgatg 1020
   ctggataacc acgctttata tgacaagacc aagagggtat ttctcaaagc ggaaagtgaa 1080
   gaagaaatct ttgcacatct gggattggac tacattgaac catgggaaag aaatgcttaa 1140
   gcttgcgc 1148
<210> 10
   <211> 379
   <212> PRT
   <213> Calf thymus
<400> 10
   Met Arg Gly Ser His His His His His His Lys Thr Pro Pro Leu Ala
   1               5                   10                  15
   Val Lys Lys Ile Ser Gln Tyr Ala Cys Gln Arg Lys Thr Thr Leu Asn
               20                  25                  30
   Asn Tyr Asn His Ile Asp Ala Phe Glu Ile Leu Ala Glu Asn Ser Glu
           35                  40                  45
   Phe Lys Glu Asn Glu Val Ser Tyr Val Thr Phe Met Arg Ala Ala Ser
       50                  55                  60
   Val Leu Lys Ser Leu Pro Phe Thr Ile Ile Ser Met Lys Asp Thr Phe
   65                  70                  75                  80
   Thr Glu Gly Ile Pro Cys Leu Gly Asp Lys Val Lys Cys Ile Ile Glu
                   85                  90                  95
   Glu Ile Ile Glu Asp Gly Glu Ser Ser Glu Val Lys Ala Val Leu Asn
               100                 105                 110
   Asp Glu Arg Tyr Gln Ser Phe Lys Leu Ser Val Phe Gly Val Gly Leu
           115                 120                 125
   Lys Thr Ser Glu Lys Trp Phe Arg Met Gly Phe Thr Phe Arg Ser Leu
       130                 135                 140
   Ser Lys Ile Met Ser Asp Lys Thr Leu Lys Lys Met Gln Lys Ala Gly
   145                 150                 155                 160
   Phe Leu Tyr Tyr Glu Asp Leu Val Ser Cys Val Thr Arg Ala Glu Ala
                   165                 170                 175
   Glu Ala Val Gly Val Leu Val Lys Glu Ala Val Trp Ala Phe Leu Pro
               180                 185                 190
   Asp Ala Phe Val Thr Met Thr Gly Gly Phe Arg Arg Gly Lys Lys Ile
           195                 200                 205
   Gly His Asp Val Asp Phe Leu Ile Thr Ser Pro Gly Ser Ala Glu Asp
       210                 215                 220
   Glu Glu Gln Leu Leu Pro Lys Val Ile Asn Leu Trp Glu Lys Lys Gly
   225                 230                 235                 240
   Leu Leu Leu Tyr Tyr Asp Leu Val Glu Ser Thr Phe Glu Lys Phe Lys
                   245                 250                 255
   Phe Thr Leu Pro Ser Arg Gln Val Asp Thr Leu Asp His Phe Gln Lys
               260                 265                 270
   Cys Phe Leu Ile Leu Lys Leu His His Gln Arg Val Asp Ser Ser Lys
   275 280 285
   Ser Asn Gln Gln Glu Gly Lys Thr Trp Lys Ala Ile Arg Val Asp Leu
   290 295 300
   Val Met Cys Pro Tyr Glu Asn Arg Ala Phe Ala Leu Leu Gly Trp Thr
   305 310 315 320
   Gly Ser Arg Gln Phe Glu Arg Asp Ile Arg Arg Tyr Ala Thr His Glu
   325 330 335
   Arg Lys Met Met Leu Asp Asn His Ala Leu Tyr Asp Lys Thr Lys Arg
   340 345 350
   Val Phe Leu Lys Ala Glu Ser Glu Glu Glu Ile Phe Ala His Leu Gly
   355 360 365
   Leu Asp Tyr Ile Glu Pro Trp Glu Arg Asn Ala
   370 375
<210> 11
   <211> 1121
   <212> DNA
   <213> Calf thymus
<400> 11
   atgagaggat cgcatcacca tcaccatcac atctcccagt acgcgtgtca aagaaaaacc     60
   actttgaaca actataacca catattcacg gatgcctttg agatactggc tgaaaattct    120
   gagtttaaag aaaatgaagt ctcttatgtg acatttatga gagcagcttc tgtacttaaa    180
   tctctgccat tcacaatcat cagtatgaag gatacagaag gaattccctg cctgggggac    240
   aaggtgaagt gtatcataga ggaaattatt gaagatggag aaagttctga agttaaagct    300
   gtgttaaatg atgaacgata tcagtccttc aaactcttta cttctgtttt tggagtggga     360
   ctgaagacat ctgagaaatg gttcaggatg gggttcagat ctctgagtaa aataatgtca     420
   gacaaaaccc tgaaattcac aaaaatgcag aaagcaggat ttctctatta tgaagacctt    480
   gtcagctgcg tgaccagggc cgaagcagag gcggttggcg tgctggttaa agaggctgtg     540
   tgggcatttc tgccggatgc ctttgtcacc atgacaggag gattccgcag gggtaagaag     600
   attgggcatg atgtagattt tttaattacc agcccaggat cagcagagga tgaagagcaa    660
   cttttgccta aagtgataaa cttatgggaa aaaaagggat tacttttata ttatgacctt    720
   gtggagtcaa catttgaaaa gttcaagttg ccaagcaggc aggtggatac tttagatcat    780
   tttcaaaaat gctttctgat tttaaaattg caccatcaga gagtagacag tagcaagtcc    840
   aaccagcagg aaggaaagac ctggaaggcc atccgtgtgg acctggttat gtgcccctac    900
   gagaaccgtg cctttgccct gctaggctgg actggctccc ggcagtttga gagagacatc    960
   cggcgctatg ccacacacga gcggaagatg atgctggata accacgcttt atatgacaag   1020
   accaagaggg tatttctcaa agcggaaagt gaagaagaaa tctttgcaca tctgggattg   1080
   gactacattg aaccatggga aagaaatgct taagcttgcg c                       1121
<210> 12
   <211> 370
   <212> PRT
   <213> Calf thymus
<400> 12
   Met Arg Gly Ser His His His His His His Ile Ser Gln Tyr Ala Cys
   1 5 10 15
   Gln Arg Lys Thr Thr Leu Asn Asn Tyr Asn His Ile Asp Ala Phe Glu
   20 25 30
   Ile Leu Ala Glu Asn Ser Glu Phe Lys Glu Asn Glu Val Ser Tyr Val
   35 40 45
   Thr Phe Met Arg Ala Ala Ser Val Leu Lys Ser Leu Pro Phe Thr Ile
   50 55 60
   Ile Ser Met Lys Asp Thr Phe Thr Glu Gly Ile Pro Cys Leu Gly Asp
   65 70 75 80
   Lys Val Lys Cys Ile Ile Glu Glu Ile Ile Glu Asp Gly Glu Ser Ser
   85 90 95
   Glu Val Lys Ala Val Leu Asn Asp Glu Arg Tyr Gln Ser Phe Lys Leu
   100 105 110
   Ser Val Phe Gly Val Gly Leu Lys Thr Ser Glu Lys Trp Phe Arg Met
   115 120 125
   Gly Phe Thr Phe Arg Ser Leu Ser Lys Ile Met Ser Asp Lys Thr Leu
   130 135 140
   Lys Lys Met Gln Lys Ala Gly Phe Leu Tyr Tyr Glu Asp Leu Val Ser
   145 150 155 160
   Cys Val Thr Arg Ala Glu Ala Glu Ala Val Gly Val Leu Val Lys Glu
   165 170 175
   Ala Val Trp Ala Phe Leu Pro Asp Ala Phe Val Thr Met Thr Gly Gly
   180 185 190
   Phe Arg Arg Gly Lys Lys Ile Gly His Asp Val Asp Phe Leu Ile Thr
   195 200 205
   Ser Pro Gly Ser Ala Glu Asp Glu Glu Gln Leu Leu Pro Lys Val Ile
   210 215 220
   Asn Leu Trp Glu Lys Lys Gly Leu Leu Leu Tyr Tyr Asp Leu Val Glu
   225 230 235 240
   Ser Thr Phe Glu Lys Phe Lys Phe Thr Leu Pro Ser Arg Gln Val Asp
   245 250 255
   Thr Leu Asp His Phe Gln Lys Cys Phe Leu Ile Leu Lys Leu His His
   260 265 270
   Gln Arg Val Asp Ser Ser Lys Ser Asn Gln Gln Glu Gly Lys Thr Trp
   275 280 285
   Lys Ala Ile Arg Val Asp Leu Val Met Cys Pro Tyr Glu Asn Arg Ala
   290 295 300
   Phe Ala Leu Leu Gly Trp Thr Gly Ser Arg Gln Phe Glu Arg Asp Ile
   305 310 315 320
   Arg Arg Tyr Ala Thr His Glu Arg Lys Met Met Leu Asp Asn His Ala
   325 330 335
   Leu Tyr Asp Lys Thr Lys Arg Val Phe Leu Lys Ala Glu Ser Glu Glu
   340 345 350
   Glu Ile Phe Ala His Leu Gly Leu Asp Tyr Ile Glu Pro Trp Glu Arg
   355 360 365
   Asn Ala
   370